(19) Europäisches Patentamt
European Patent Office
Office européen des brevets

(11) Publication number : **0 562 765 A2**

(12) **EUROPEAN PATENT APPLICATION**

(21) Application number : **93302065.3**

(51) Int. Cl.$^5$ : **C12Q 1/68**

(22) Date of filing : **18.03.93**

(30) Priority : **25.03.92 US 857205**

(43) Date of publication of application :
**29.09.93 Bulletin 93/39**

(84) Designated Contracting States :
**AT BE CH DE DK ES FR GB GR IE IT LI LU NL PT SE**

(71) Applicant : **ELI LILLY AND COMPANY**
**Lilly Corporate Center**
**Indianapolis Indiana 46285 (US)**

(72) Inventor : **Meier, Timothy Ivan**
**10234 Breckenridge Avenue**
**St. Ann, Missouri 63074 (US)**
Inventor : **Yao, Raymond Che-Fong**
**1189 Woodgate Drive**
**Carmel, Indiana 46032 (US)**

(74) Representative : **Tapping, Kenneth George et al**
**Erl Wood Manor**
**Windlesham Surrey, GU20 6PH (GB)**

(54) **Rapid assay for detection of nucleic acid-binding factors.**

(57) This invention consists of methods for the detection of nucleotide-binding compounds in complex fluid mixtures. The invention consists of fixing a target polynucleotide of known sequence to a non-porous support which comprises or is contained within a container. The challenging sample fluid is then introduced in such a way that compounds present in the fluid bind to the target polynucleotide. This binding blocks the hybridization of a second, complementary polynucleotide to which is attached a label comprising a moiety capable of generating a detectable signal. The invention further provides for indirect means of binding the target polynucleotide to the support and of generating the detectable signal.

EP 0 562 765 A2

Methods for detecting and identifying substances that bind to nucleic acids comprise one of the burgeoning fields of biology, crossing the boundaries between scientific fields often considered distinct. For example, the clinical microbiologist can use such techniques for the rapid identification of pathogenic bacteria, parasites, fungi, and viruses. Environmental scientists can use these techniques for the rapid identification of contamination of groundwater or potable water with sewage or chemical runoff. The molecular biologist or biochemist traditionally uses these techniques as assay methods while purifying proteins which bind to nucleic acids, in either a site-specific manner or as general nucleic acid-binding compounds.

Traditional methods for detecting and identifying bacteria in a complex fluid vary with the type of sample and the suspected pathogen. No currently available method is optimal for the detection of all pathogens. Often, a combination of methods must be employed to winnow the possibilities, a tedious and lengthy process. Many disease organisms require very fastidious culture conditions and grow at an exceptionally slow rate. For example, it routinely requires three to four weeks for the clinical microbiologist to grow enough of the organism Mycobacterium tuberculosis, the causative agent of tuberculosis, to perform many of the tests required to identify and treat the disease. R. Weiss, Science, 255:148-150 (1992). There are no currently available techniques for the rapid identification of the organism.

Current cultivation methods suffer from a number of very serious shortcomings including: high material costs; labor intensive; the extensive handling of potentially dangerous bodily fluids; false positives due to this extensive handling; false negatives due to fastidious media conditions or low viability of sample; relatively long time for positive diagnosis and identification; and lack of flexibility of the testing procedures. The techniques described in the instant invention provide rapid, reproducible methods for identification of microorganisms. These methods also provide the skilled practitioner with the flexibility to design the testing regimen as the practitioner, not the marketer, deems appropriate.

Biochemists attempting to purify proteins that bind to a specific sequence of DNA or RNA are often confronted with difficult assays, resulting in false positives or negatives, and which generally consume a great deal of time. These assays force the practitioner to store all samples and hesitate before designing and beginning the next purification step. Techniques such as nitrocellulose filter binding, DNase I footprinting, methylation interference, and gel mobility shift, require DNA probes radioactively labeled with a very high specific activity and can be very difficult for the novice.

The instant invention describes techniques for the rapid identification of fluids containing factors which bind to nucleic acids, either in a sequence-specific manner, or in a general fashion, depending on the needs and designs of the practitioner. These techniques can be performed without the use of radioisotopes, which are dangerous to handle, result in auto-degradation of the probe, and have a relatively short half life.

This invention describes methods for the detection of nucleotide-binding compounds in complex fluid mixtures. One embodiment of the invention entails, first, fixing a target polynucleotide of known sequence to a non-porous support which comprises or is contained within a container system. The challenging sample fluid is then introduced in such a way that compounds present in the fluid bind to the target polynucleotide. This binding blocks the subsequent hybridization of a second, complementary polynucleotide to which is attached a label comprising a moiety capable of generating a detectable signal. The signal is then generated, and the diminution of the signal from experimental sections, relative to the control sections, is detected.

A further embodiment of this invention employs a ligand/receptor pair which mediates the binding of the target polynucleotide to the support. Additional embodiments of this invention employ a second ligand/receptor pair that mediates the binding of the signaling moiety and the complementary polynucleotide.

The instant invention describes methods for the identification of compounds from complex fluids that bind to a polynucleotide sequence, either in a sequence-specific manner or as general nucleotide-binding factors. Examples of these complex fluids include: blood or serum; cerebrospinal fluids; lymphatic fluids; urine; cell extracts; fermentation broths; drinking water; sewage; synovial fluid; pharmaceuticals; and foods and beverages.

The methods of the instant invention can be employed to determine the presence of general nucleotide-binding proteins, sequence-specific nucleotide-binding proteins, intercalating agents, ribonucleic acids, or oligonucleotides in a fluid. The methods of the instant invention can be employed by practitioners in many broad areas of practice. The techniques are similar regardless of the field, the only major difference being the type of complex fluid used.

One broad area for which this invention can be used is the identification of living organisms or residues from these organisms in fluids. For example, the presence of undesired bacteria in fluids can be readily determined.

These techniques employ probes which are usually based upon ribosomal ribonucleic acid (rRNA) sequences found in the bacteria. Ribosomes are generally present in about 20,000 copies per cell, constituting about a fourth of the mass of a cell. L. Stryer, Biochemistry (3d ed. 1988). Each of the three ribosomal RNAs

is present as one copy per ribosome. Id.

Some segments of the rRNAs, particularly segments of the rRNA known as 16S, are universal to all living organisms and can, therefore, be used as universal probes, i.e. to determine the presence of any organic matter in a fluid. D.J. Lane, et al., Proceedings of the National Academy of Sciences, U.S.A., 82:6955 (1985). Phylogenetic group-specific probes have been described which can be readily employed to distinguish between bacteria of different phylogenetic groups. S.J. Giovannoni, et al., Journal of Bacteriology, 170:720-726 (1988); D. J. Lane, et al., European Patent Publication WO 90/15157, published December 13, 1990. For example, the presence of colicins or pathogenic bacteria in drinking water can be rapidly determined using probes specific for the colicins or pathogens.

Because of the versatility of the methods described in the instant invention, the level of stringency can be dictated by the practitioner of the invention, almost without limitation. Depending upon the desires of the practitioner, different types of probes can be used to differentiate organisms based on any level of taxonomic differences from the broadest [for example, the Urkingdoms described by C. Woese, Microbiological Reviews, 51:221-271 (1987)] to the most specific (mutants of a particular subspecies).

Another broad field in which the instant invention can be utilized is in the area of protein purification, especially those proteins which bind to nucleic acids. The techniques disclosed in this invention provide rapid, sensitive assays to be used in studying, for example, fractions from column chromatography. These techniques provide much more rapid results than such traditional assays as DNase I footprinting, gel mobility shifting, and nitrocellulose filter binding. Additionally, these techniques can be performed without the use of radioisotopes.

The methods of the instant invention can also be used to detect specific proteins (e.g. c-myc) in bodily fluids which may serve as an indicator of the presence of neoplasms. These methods can also be used to rapidly screen biological cultures for the presence or absence of a particular factor. The skilled practitioner, knowing the desires of the experiment can tailor the experimental and control sections so as to provide almost unlimited flexibility.

The general methodology of this invention first entails the immobilization of a target polynucleotide to a solid support which comprises or is contained within a container system. Such support can be particles within a container or the surfaces of the container itself. The characteristics of the container system employed depend upon the type of signaling moiety used. For example, the use of a radioactive signal requires the container to be pervious to the emitted particle. When chromogenic or fluorogenic signals are used, the container system must either be transparent at the wavelength employed to detect said signal or it must be constructed in such a way as to allow a direct viewing of the reaction fluid.

The preferred system employs a plate of wells, such as those commonly used for microtiter assays or tissue culture. The most preferred system for chromogenic or fluorogenic signals employs those plates which have 96 wells, have flat bottoms to the wells, and are pre-treated so as to accept binding to the well surfaces readily.

The affixation of the target polynucleotide to the support can be either a direct binding of the polynucleotide to the support surface or may be mediated by ligand/receptor interaction. The challenging fluid is then introduced into the experimental sections of the container system containing the bound target. Negative control sections are those to which no challenging fluid is added.

The presence of any compounds in that fluid capable of binding to the target polynucleotide sequence will result in a complexing of the compound to said target.

The presence of such compounds in the challenging fluid is determined by attempting to hybridize a second, complementary polynucleotide to the target polynucleotide. The complex of the compound fund to the target polynucleotide results in a diminution of the hybridization of the target polynucleotide and its complement relative to the controls in which no challenging fluid is added.

The amount of hybridization of the target polynucleotide and its complement is determined by a signal moiety attached, either directly or indirectly, to the complementary polynucleotide. Upon generation of the signal, the efficiency of the hybridization is readily determined. Diminution in that efficiency is indicative of the presence of a compound in the challenging fluid blocking hybridization.

The target polynucleotides selected for these assays can be deoxyribonucleic acids or ribonucleic acids of any length. The exact sequences and lengths of the polynucleotides employed in the assay are left to the discretion of the technician, depending upon the desired objectives of the assay. This flexibility allows the skilled practitioner to run multiple assays simultaneously, resulting in greater efficiency.

The techniques for synthesizing polynucleotides of a desired sequence are well-known in the art. For example, a known template nucleic acid can be readily reproduced enzymatically by a large number of techniques familiar to those skilled in the art. [See, e.g., J. Sambrook, et al., Molecular Cloning: A Laboratory Manual, (2d ed. 1989); F.M. Ausubel, et al., Current Protocols in Molecular Biology, (1987 and supplements thereof)]

A preferred embodiment of this invention employs chemically synthesized oligonucleotides which are prepared by techniques familiar to those skilled in the art. [See, e.g., M.J. Gait, ed., Oligonucleotide Synthesis,

A Practical Approach, (1984).] Chemically synthesized oligonucleotides are readily available from commercial sources as well. It is generally preferred that oligonucleotides of 20 residues or less be employed.

An especially preferred embodiment of this invention employs oligonucleotides which are resistant to nucleases. One such embodiment uses oligonucleotides which are linked through phosphorothioate linkages, rather than the biologically typical phosphodiester linkages. These oligonucleotides have the general structure:

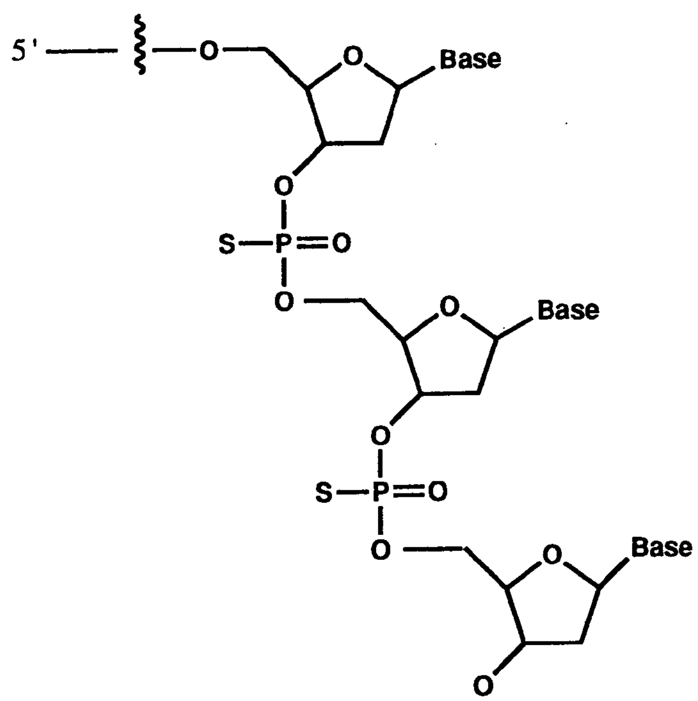

Oligonucleotides with phosphorothioate linkages are known to be less susceptible to nuclease activity but still bind normally to most compounds. C.A. Stein, et al., Nucleic Acids Research, 16:3209-3221 (1988).

Two methods are commonly used to synthesize phosphorothioate oligodeoxynucleotides. The first is through the use of the normal beta-cyanoethyl phosphoramadite synthesis scheme coupled with mild oxidation of the phosphite triester intermediate with elemental sulfur at each cycle of synthesis. W.J. Stec, et al., Journal of the American Chemical Society, 106:6077-6079 (1984). A second protocol utilizes H-phosphonate chemistry with strong global sulfur oxidation of the final oligonucleotide at the completion of the oligonucleotide synthesis. P.J. Garegg, et al., Tetrahedron Letters, 27:4055-4058 (1986). Phosphorothioated oligoribonucleotides can also be readily synthesized using H-phosphonate chemistry. S. Agrawal and J. Y. Tang, Tetrahedron Letters, 31:7541-7544 (1990).

The polynucleotides can be affixed to the support surfaces either directly or indirectly. In the direct method of fixation, solutions containing the target polynucleotides are added to the appropriate activated supports. The binding reaction is incubated, usually overnight, at 4°C. The excess activated binding sites of the supports are then saturated by adding a solution containing a relatively inert protein, such as bovine serum albumin. This saturation reaction retards non-specific binding of the signal-generating moiety later, thereby reducing background signal. The result of the binding reaction is the target polynucleotides being tightly affixed to the surfaces of the supports.

The indirect method of affixing the polynucleotide employs a ligand/receptor reaction. A ligand is bound to the support surfaces. The receptor, covalently linked to the target polynucleotide, is then introduced into the containers. The ligand/receptor interactions then serve to affix the polynucleotides to the support surfaces. Preferred ligand/receptor pairs include biotin and streptavidin, biotin and avidin, and sugars and lectins.

The use of sugar/lectin pairs can be especially valuable when the polynucleotides have been heavily modified by a host organism. For example, naturally occurring DNA of the bacteriophage T4 grown in most strains of Escherichia coli is heavily glucosylated. E.A. Birge, Bacterial and Bacteriophage Genetics, (1981) p. 69-72. Binding of the lectin (phytohemagglutinin) concanavalin A, which tightly binds to glucopyranosyl residues, to the support surfaces allows the efficient binding of the modified polynucleotide.

The use of biotin and avidin or biotin and streptavidin as the ligand/receptor pair is generally preferred because of their extremely high affinity interaction ($K_d = 10^{-15}$). Avidin is a glycoprotein which naturally exists as a tetramer containing four identical subunits, each with a molecular weight of 15,000 daltons. It is readily isolatable from egg whites. See, e.g., M. Wilchek and E.A. Bayer, Analytical Biochemistry, 171:1-32 (1988) and references therein.

An especially preferred embodiment of this invention employs streptavidin in place of egg-white avidin. Streptavidin is often preferred over avidin for several reasons. Streptavidin, isolated from culture filtrates of a Streptomyces species, differs from avidin in having no carbohydrate moieties and a pl of 5.0, compared to an avidin pl of 10.0. This is important because the presence of carbohydrates and high pl values can result in increased non-specific binding. A deglycosylated form of egg-white avidin may also be used to decrease background binding. Y. Hiller, et al., Biochemical Journal, 248:167-171 (1987).

The synthesis of biotinylated nucleotides was first described by Langer et al. in 1981. P.R. Langer, et al., Proceedings of the National Academy of Sciences, USA, 78:6633-6637 (1981). Since that time, a multitude of methods have been developed for the incorporation of biotinylated nucleotides in polynucleotides, both enzymatically and chemically. See, Wilchek and Bayer, supra. Generally, the biotin is covalently attached to the C5 position of the pyrimidine ring by way of an allylamine linker arm. Biotinylated oligonucleotides are commercially available from a number of sources.

The challenging fluid, the complex fluid to be tested, may be pre-treated, depending upon the characteristics of the factors sought. For example, if the test is designed to detect small molecules, the fluid may be filtered through a membrane with pores that retain molecules below a specified molecular weight. If the factor sought is heat-stable, the sample fluid may be heated or boiled to denature proteins and destroy heat-labile compounds which may add to the background. If the skilled practitioner knows the chemical characteristics of the factor being sought, that practitioner may treat the sample so as to decrease non-specific interactions which can increase background.

The temperature at which the hybridization of the complementary polynucleotide to the target polynucleotide is performed depends in part upon the characteristics of the compound sought. The hybridization reaction is generally performed at temperatures from about 4°C to about 60°C. The length of the shorter of the target polynucleotide and the complementary polynucleotide as well as its base composition is generally determinative of the maximum temperature permissible.

When short oligonucleotides are used for the target or complementary polynucleotide, the melting temperature of the oligonucleotide can be approximated by the formula:

$$T_m = 2(A + T) + 4(G + C)$$

wherein $T_m$ refers to the melting temperature in degrees Celsius, (A + T) corresponds to the number of adenine and thymine residues, and (G + C) corresponds to the number of guanine and cytosine residues in the oligonucleotide. This melting temperature serves as the maximum temperature at which the hybridization can be performed.

The complementary polynucleotide sequence has attached to it a moiety capable of generating a signal. The signaling moiety can be attached to the complementary polynucleotide sequence either directly or indirectly.

Direct, covalent attachment of the signaling moiety to the complementary polynucleotide sequence enables the practitioner to save a single step and is of advantage when a number of tests are being performed with one complementary polynucleotide and one preferred signal. If, however, the practitioner desires to employ multiple complementary polynucleotide sequences or multiple signaling moieties, said technician may find it more advantageous to employ an indirect method of signal generation.

The method of directly attaching the signaling moiety to the complementary polynucleotide sequence depends upon the particular moiety desired. A preferred embodiment of this invention employs enzymes coupled to the complementary polynucleotides, said enzymes capable of generating a chromogenic signal when the appropriate substrate is introduced.

As an example, the enzyme alkaline phosphatase (EC 3.1.3.1) can be easily linked to a complementary oligonucleotide containing a reactive thiol group. Such oligonucleotides are readily available from commercial sources. The alkaline phosphatase is generally conjugated to the oligonucleotide through a heterobifunctional cross-linker such as 4-(N-maleimidomethyl)cyclohexane-1-(carboxylic acid-N-hydroxysuccinimide ester. S. Yoshitake, et al., Journal of Biochemistry, 92:1413 (1982). Enzymes can be linked to polynucleotides other than chemically synthesized oligonucleotides by established procedures. Id.

The signal is generated by the addition of a chromogenic substrate such as p-nitrophenyl phosphate (PNP). Hydrolysis of PNP results in release of the p-nitrophenol group, causing an increase in the absorbance at 405 nm ($A_{405}$) and appearance of a yellow color.

Other examples of preferred enzymes capable of generating a chromogenic signal are: horseradish peroxidase, β-D-glucoronidase, β-D-galactosidase, 6-phospho-β-D-galactoside 6-phosphogalactohydrolase, α-D-galactosidase, β-D-glucosidase, α-amylase, α-glucosidase, neuraminadase, butyrate esterase, lipases, acid phosphatase, pyroglutamyl aminopeptidase, L-alanine aminopeptidase, arylaminopeptidases, coagulose, urease, luciferase, glucose oxidase, and other peroxidases.

The substrate employed as well as the wavelength at which the absorbance is read is determined by the particular enzyme used to generate the signal. The type of enzyme employed may be determined in part by the type of factor sought. For example, phosphodiesterases or nucleases are generally not employed as they can result in digestion of the target and complementary polynucleotides. Similarly, if a protein is believed to be involved in the complexing to the target polynucleotide, it may be unwise to use a peptidase.

Other preferred means of generating a signal involve the use of fluorogenic substrates, radioactive signals and electrical or magnetic signals. Fluorogenic substrates conjugated (directly or indirectly) to the complementary polynucleotide provide a rapid means of quantifying the hybridizing polynucleotide. Examples of fluorogenic substrates include fluorescein isothiocyanate, Texas Red, rhodamine isothiocyanate, sulforhodamine, luciferase, eosin isothiocyanate, dansyl chloride, trifluoromethylcoumarin, phthaldialdehyde, quinolizino-substituted fluorescein isothiocyanate, and tetramethylrhodamine isothiocyanate. A fluorochrome can be incorporated into a polynucleotide through an allylamine linker arm, often binding to the C5 position of the pyrimidine ring.

Rapid quantitation of the fluorescence can be performed using a commercially-available fluorometer (spectrofluorometer) capable of reading microtiter plates. The wavelengths of the emitting light and the receiver filter depend upon the particular fluorogenic substrate employed.

Preferred examples of radioisotopes used as signaling moieties are phosphorous-32, iodine-125, carbon-14, tritium, and sulfur-35. The method of linking the radioactive signal to the complementary polynucleotide depends upon the radioisotope employed and whether there is direct or indirect attachment of the signaling moiety and the complementary polynucleotide. For example, phosphorous-32 can be integrated directly into the complementary polynucleotide by the use of a kinasing reaction. T. Maniatis, et al., Molecular Cloning, A Laboratory Manual, pp. 123-125 (1982).

Indirect methods of linking the signaling moiety and the complementary polynucleotide employ the use of ligand/receptor interactions. The type of ligand and receptor pair employed will be determined, in part, by whether a ligand/receptor pair was employed in the binding of the target polynucleotide to the surfaces of the test system. The use of the same ligand/receptor pair as used earlier could result in high levels of background.

Preferred ligand and receptor pairs for the binding of the signaling moiety to the complementary polynucleotide are: avidin and biotin, streptavidin and biotin, lectins and sugars, antigens and antibodies, and receptors and hormones. One especially preferred ligand and receptor pair for the binding of the complementary polynucleotide and the signaling moiety is digoxigenin and anti-digoxigenin antibody. Like biotin, digoxigenin is attached to the C5 position of the pyrimidine ring via a linker. Digoxigenin-11-dUTP may be incorporated into DNA by nick translation or random primed synthesis. Ausubel, et al., supra at 3.18.4.

Anti-digoxigenin antibody covalently conjugated to a signaling moiety is then used to create an indirect binding of the signaling moiety to the complementary polynucleotide mediated by the digoxigenin/antibody conjugate. The signaling moiety is bound to the antibody in much the same way as the signaling moiety can be bound to the polynucleotide. For example, the enzyme horseradish peroxidase can be linked to the antibody by way of a heterobifunctional cross-linker. The generation of the signal is performed as when the signaling moiety is directly linked to the complementary polynucleotide.

The following examples further illustrate the methods employed in this invention. The examples are provided for purposes of illustration only and are not to be construed as limiting the scope of the instant invention in any way.

The terms and abbreviations used in the instant examples have their normal meaning unless otherwise designated. For example, "°C" refers to degrees Celsius, "rRNA" refers to ribosomal ribonucleic acid, "ml" refers to milliliters, "μl" refers to microliters, "M" refers to molar or molarity, "nm" refers to nanometers, and "μg" refers to micrograms.

Example 1

Detection of Colicins in Fresh Water Using the Direct Target Binding/Direct Signal Generation Method

The target oligonucleotide is bound to the wells of a 96 well, high binding, flat bottom, microtiter plate (e.g. Costar, Northumberland, England, Cat. No. 3590) by the addition of 200 μl of a 5 ng/ml solution of the target oligonucleotide with the sequence:

5'-AGAGTTTGATCATGG-3'

in hybridization buffer [5x SSC buffer (0.75 M sodium chloride, 0.075 M sodium citrate, pH 7.0), 0.1% N-lauroylsarcosine, 0.02% sodium dodecyl sulfate]. This sequence corresponds to a region of the 16S rRNA which is conserved among many colicins. D. Lane, et al., PCT Publication WO 90/15157, published December 13, 1990. This coating reaction is allowed to proceed overnight at 4°C.

After removal, by aspiration, of the supernatant containing any unbound oligonucleotide, the unbound activated sites on the well surface are saturated by the addition of 200 μl of a 50 μg/ml solution of bovine serum albumin in hybridization buffer. This reaction is incubated an additional four hours at 4°C. The plates are then washed once with sterile distilled water to remove unbound BSA.

To each experimental well is then added 200 μl of the water to be tested. No water sample is added to each of the negative control wells. The water sample may first be filtered, either through a size exclusion membrane, or through a nitrocellulose filter which retains proteins and particulates while allowing unbound nucleic acids to pass through. The reaction of the water sample with the target oligonucleotide is incubated at room temperature for one hour. Each well is then washed once with 3x SSC buffer [0.45 M sodium chloride, 0.045 M sodium citrate (pH 7.0)] to remove unbound materials.

The complementary oligonucleotide of the sequence:

5'-FITC-AGCCGCCGCGGTAAT-3'

covalently linked to fluorescein isothiocyanate, is introduced into the reaction by the addition to each well of 200 μl of a 2 ng/ml solution of the conjugate in hybridization buffer. This hybridization reaction is allowed to proceed for at least one hour at room temperature. Each well is then washed three times with 3x SSC buffer to remove the unhybridized oligonucleotide/fluorochrome conjugate.

The intensity of the fluorescence can be determined using a fluorometer equipped with an excitation beam of blue light and a filter of 450-490 nm. Diminution of the fluorescent signal in the experimental wells, relative to the negative control wells to which no water samples are added, is indicative of a positive result.

Example 2

Detection of Eukaryotic or Prokaryotic Organisms in a Pharmaceutical Sample Employing the Indirect Target Binding/Direct Signal Generation Method.

Each well of a 96 well, high binding, flat bottom, microtiter plate is pre-coated by the addition of 200 μl of a 15 μg/ml solution of streptavidin in 0.05 M carbonate-bicarbonate buffer (pH 9.6). This pre-coating reaction is allowed to incubate overnight at 4°C. The plates are then washed once with distilled water to remove unbound streptavidin.

The target oligonucleotide is introduced by adding to each well 200 μl of biotinylated target oligonucleotide (2 ng/ml in hybridization buffer). The sequence of the target oligonucleotide is:

5'-biotin-ATTACCGCGGCGGCT-3'

This sequence corresponds to the universal probe described in Giovannoni, et al., Journal of Bacteriology, 170:720, 722 (1988). This mixture is incubated at room temperature for one hour. Each well is then washed once with 3x SSC buffer to remove uncaptured oligonucleotide.

To each experimental well is then added 200 μl of the complex fluid, serum in this instance. Several dilutions of the serum are employed to optimize signal generation. Samples are diluted in sterile hybridization buffer. This reaction is incubated at room temperature for one hour. Each well is washed once with 3x SSC buffer.

The complementary oligonucleotide of the sequence:

5'-HRP-S-AGCCGCCGCGGTAAT-3'

is incorporated into the reaction by the addition into each well of 200 μl of a 2 ng/ml solution of the horseradish peroxidase-linked complementary oligonucleotide in hybridization buffer. The hybridization reaction is allowed to proceed for at least one hour at room temperature. Each well is then washed three times with 3x SSC to remove the unhybridized oligonucleotide/enzyme conjugate, thereby decreasing the background signal.

The signal is generated by the addition of 200 μl of o-phenylenediamine dihydrochloride substrate [40 mg OPD/100 ml phosphate-citrate buffer (25.7 ml 0.2 M dibasic sodium phosphate, 24.3 ml 0.1 M citric acid, 50 ml water, pH 5.0)] to each well. The reaction is allowed to proceed at room temperature until the desired optical density in the control reactions is reached. It is desirable for the optical density of the control to be approximately 1.0.

The reactions are halted by the addition of 50 µl of 5 N sulfuric acid. The plates are then read spectrophotometrically at 492 nm on a microtiter plate reader. Diminution of the signal from the chromogenic substrate, relative to those wells which serve as negative controls, is indicative of a positive result.

Example 3

Detection of HIV-1 Rev Protein Present in the Supernatant of Cultured Cells Using the Direct Target Binding/Indirect Signal Generation Method

The target oligoribonucleotide is bound to the wells by the addition to each well of 200 µl of a 5 ng/ml solution of the target phosphorothioated oligoribonucleotide of the sequence:

5'-AGCAGCAGGAACACUAUGGGCGCA-3'

in hybridization buffer. This sequence corresponds to the Rev response element of HIV-1 RNA. T.J. Daly, et al., Nature (London), 342:816-819 (1989); S. Daefler, et al., Proceedings of the National Academy of Sciences, USA, 87:4571-4575 (1990). If desired, 10 units, of placental RNasse inhibitor can be added to each well. This binding reaction is allowed to proceed overnight at 4°C. After removal of the supernatant containing any unbound oligonucleotide, the unbound activated sites on the well surfaces are saturated by the addition of 200 µl of a 50 µg/ml solution of bovine serum albumin in hybridization buffer, which is further incubated at 4°C for at least four hours. The plates are then washed once with sterile distilled water to remove unbound BSA.

To each well is then added 200 µl of supernatant from cultured cells. The reaction of the supernatant of the cultured cells with the target oligonueleotide is incubated at room temperature for one hour. Each well is then washed once with 3x SSC buffer to remove unbound materials.

The complementary oligodeoxynucleotide of the sequence:

5'biotin-TCGGCCCATAGTGTTCCTGCTGCT-3'

is hybridized by adding 200 µl of a 2 ng/ml solution of the biotinylated oligonucleotide in hybridization buffer to each well. The hybridization reaction is allowed to incubate at room temperature for at least one hour. Each well is then washed three times with 3x SSC to remove the unhybridized oligonucleotide/ligand conjugate.

The signal is generated by adding 200 µl of a 5 µg/ml solution of avidin conjugated to the fluorochrome Texas Red. The reaction between any bound biotin and the avidin is allowed to incubate for one hour at room temperature. Each well is then washed three times with 3x SSC buffer to remove any unbound avidin/Texas Red conjugate. The diminution of the fluorogen relative to the control sample can be quantified using a fluorometer with a 510-560 nm filter.

Example 4

Detection of Mycobacterium tuberculosis rRNA from a Sputum Sample Using the Indirect Target Binding/Indirect Signal Generation Method

A 96 well, high binding, flat bottom, microtiter plate is pre-coated by adding to each well 200 µl of a 15 µg/ml solution of streptavidin in 0.05 M carbonate-bicarbonate buffer (pH 9.6). This coating reaction is allowed to proceed overnight at 4°C. The plate is washed once with sterile distilled water to remove any unbound streptavidin.

To each well is then added 200 µl of the biotinylated oligonucleotide of the sequence:

5'-biotin-GCCCTACGTACAGAACA-3'

which is present at a concentration of 2 ng/ml in hybridization buffer. This ligand/receptor reaction is allowed to incubate at room temperature for one hour. Each well is then washed once with 3x SSC buffer to remove unbound oligonucleotides.

Two hundred microliters of sputum, serially diluted in hybridization buffer, is added to each well. This reaction is incubated at 30°C for one hour. The plate is then washed once with 3x SSC buffer.

The complementary polynucleotide of the sequence:

5'-TG-digU-TC-digU-G-digU-ACG-digU-AGGGC-3'

in which digoxigenin coupled via an 11 atom spacer to uridine triphosphate (digU) is incorporated in place of

some of the thymidine triphosphate, is introduced into the reaction by the addition of 200 µl of a 5 ng/ml solution of the substituted polynucleotide in hybridization buffer. The hybridization reaction is allowed to incubate at 45°C for at least one hour. The wells are then washed three times with 3x SSC buffer.

The signaling moiety is entered into the reaction by the addition of anti-digoxigenin antibody (Fab fragments) covalently linked to alkaline phosphatase. Two hundred microliters (2 µg) of the anti-digoxigenin antibody/alkaline phosphatase conjugate in phosphate buffered saline is then added to each well. The antibody/antigen coupling reaction is allowed to incubate for one hour at room temperature. The wells are then washed once with 3x SSC buffer to remove unbound antibody/enzyme conjugate.

The signal is generated by the addition of 200 µl of the substrate of alkaline phosphatase, p-nitrophenyl phosphate (3 mM NPP in 0.05 M sodium carbonate and 0.05 mM magnesium chloride). This hydrolysis reaction is incubated at room temperature for at least one hour or until controls reach the desired optical density (about O.D. 1.0). The reaction is then terminated by the addition of 25 µl of 0.5 M sodium hydroxide. The wells are read spectrophotometrically at 405 nm. Diminution of the signal from the chromogenic substrate, relative to those wells which serve as negative controls, is indicative of a positive result.

## Claims

1. A method of detecting compounds that bind to a target polynucleotide which comprises:
   a. fixing said target polynucleotide to a solid support which comprises or is contained within a container system;
   b. forming an entity comprising said target polynucleotide bound by a compound which blocks hybridization of a second polynucleotide, complementary to the target polynucleotide, said second polynucleotide having attached a label moiety capable of generating a detectable signal;
   c. generating said signal; and
   d. detecting reduction in said signal relative to a control.

2. A method of detecting compounds that bind to a target polynucleotide which comprises:
   a. fixing a receptor to a solid support which comprises or is contained within a container system;
   b. complexing to said receptor a target polynucleotide bound to a ligand recognized by the receptor;
   c. forming an entity comprising said target polynucleotide bound by a compound which blocks hybridization of a second polynucleotide, complementary to the target polynucleotide, said second polynucleotide having attached a label moiety capable of generating a detectable signal;
   d. generating said signal; and
   e. detecting reduction in said signal relative to a control.

3. A method of detecting compounds that bind to a target polynucleotide which comprises:
   a. fixing a receptor to a solid support which comprises or is contained within a container system;
   b. complexing to said receptor a target polynucleotide bound to a ligand recognized by the receptor;
   c. forming an entity comprising said target polynucleotide sequence bound by a compound which blocks hybridization of a second polynucleotide complementary to the target polynucleotide, said second polynucleotide having attached a second ligand capable of binding to a second receptor;
   d. forming a complex between the second ligand and the second receptor, said second receptor having attached a label moiety capable of generating a detectable signal;
   e. generating said signal; and
   f. detecting reduction in said signal relative to a control

4. A method of detecting compounds that bind to a target polynucleotide which comprises:
   a. fixing said target polynucleotide to a solid support which comprises or is contained within a container system;
   b. forming an entity comprising said target polynucleotide sequence bound by a compound which blocks hybridization of a second polynucleotide complementary to the target polynucleotide, said second polynucleotide having attached a ligand capable of binding to a receptor;
   c. forming a complex between the ligand and the receptor, said receptor having attached a label moiety capable of generating a detectable signal;
   d. generating said signal; and
   e. detecting reduction in said signal relative to a control.

5. A method as claimed in any one of Claims 1 to 4, wherein said target polynucleotide is a polydeoxyribonucleotide.

6. A method as claimed in any one of Claims 1 to 4, wherein said target polynucleotide is a polyribonucleotide.

7. A method as claimed in any one of Claims 1 to 4, wherein said signal is detected employing spectrophotometric techniques.

8. A method as claimed in any one of Claims 1 to 4, wherein said signal is detected employing radioactive techniques.

9. A method as claimed in any one of Claims 1 to 4, wherein said signal is detected employing electrical or magnetic techniques.

10. A method as claimed in any one of Claims 1 to 4, wherein said signal is detected employing fluorescent techniques.